# EUROPEAN PATENT APPLICATION

(11) **EP 1 637 161 A1**
(43) Date of publication of application: **22.03.2006**
(21) Application number: 04727760.3
(22) Date of filing: 15.04.2004
(51) Int. Cl.: A61K 45/00, A61K 31/192, A61K 31/426, A61P 25/28, A61P 25/16, A61P 9/10, A61P 21/00, A61P 25/00

(54) **REMEDY FOR CEREBRAL NEURODEGENERATIVE DISEASES USING PPAR&dgr; AGONIST**

(30) Priority: 22.04.2003 JP 2003117381
(71) Applicant: Astellas Pharma Inc., Tokyo 103-8411 (JP)
(72) Inventor: KITA, Yasuhiro,, Handa-shi, Aichi 475-0878 (JP); YAMAZAKI, Takao, c/o Astellas Pharma Inc., Chuo-ku, Tokyo 103-8411 (JP); MURAMOTO, Masakazu, c/o Astellas Pharma Inc., Chuo-ku, Tokyo 103-8411 (JP); IWASHITA, Akinori, c/o Astellas Pharma Inc., Chuo-ku, Tokyo 103-8411 (JP); MORIGUCHI, Akira, c/o Astellas Pharma Inc., Chuo-ku, Tokyo 103-8411 (JP); MATSUOKA, Nobuya, c/o Astellas Pharma Inc., Chuo-ku Tokyo 103-8411 (JP)
(74) Representative: Gille Hrabal Struck Neidlein Prop Roos
(86) International application number: PCT/JP2004/005429
(87) International publication number: WO 2004/093910

(57) **Abstract**

In accordance with the invention, a compound with a protective action for nerve cell can be reselected by adding PPARδ agonist to a culture cell system where toxic substances such as thapsigargin, MPP⁺ and staurosporine are preliminarily allowed to react and reselecting a compound improving the survival rate. The compound selected by such method can be used as an active ingredient of a therapeutic agent for neurodegenerative diseases such as cerebral infarction and Parkinson's disease. Thus, the invention is very useful for research works for creating novel pharmaceutical agent.

## Description

### Technical Field

The present invention relates to the use of a compound with a PPARδ agonist activity as a therapeutic agent for cerebral neurodegenerative diseases. Additionally, the invention relates to a method for therapeutically treating cerebral neurodegenerative diseases which comprises administering a pharmaceutical agent containing the compound as an active ingredient.

### Background of the Invention

Various central diseases such as cerebral infarction, Parkinson's disease, Alzheimer's disease and Huntington's disease are all caused by the degeneration of nerve cells and trigger various severe disorders.

As well known, cerebral tissues are damaged by for example the occlusion of cerebral blood tubes, resulting in no blood flow (cerebral infarction) and the rupture of cerebral blood tubes, leading to bleeding (cerebral hemorrhage), so that blood flow enough for cerebral nerve cells to be viable cannot be securely retained. Accordingly, brain falls into necrosis in a very short time. The death of nerve cells following such ischemia starts from the necrosis of nerve cells in the core region of an infarct lesion directly exposed to an ischemic state and spreads gradually to the penumbra, so that damages spread to a great number of nerve cells. Among them, the necrosis of the core region is caused irreversibly in such a very short time that it is considered that substantial treatment thereof is impossible. Because the death of nerve cells in the penumbra gradually progresses under the influence of the necrosis of adjacent nerve cells, however, it is expected that the death thereof may possibly be suppressed in a reversible manner at some extent at the initial stage. Therefore, various attempts have been made in such expectation to develop various therapeutic agents. Currently, however, the molecular mechanism of the degeneration of nerve cells in such ischemic state has not yet been sufficiently elucidated. No pharmaceutical agent with a clearly verified therapeutic effect on the suppression of the death of nerve cells on clinical site has been found.

Meanwhile, Parkinson's disease (PD) is a central disease caused by gradual degeneration of nigrostriatal dopaminergic neurons over a long time, which causes symptoms such as tremor, muscular rigor and akinesia. The disease is a disease at a high incident rate per population but the detailed etiology causing the onset has not yet been elucidated. It is supposed that genetic factors, endogenous and exogenous toxins, oxidative stress and the like may subtly give an influence on the exacerbation of the disordered conditions. In this disease, only nigrostriatal dopaminergic neurons are selectively degenerated among numerous nerve cells. Not any pharmaceutical agent with a clinically clearly certified potency to significantly suppress the neurodegeneration to substantially suppress the progress of Parkinson's disease itself has existed yet.

Therefore, the development of a pharmaceutical agent suppressing neurodegeneration or a pharmaceutical agent promoting the regeneration of damaged nerves has been strongly desired so as to essentially treat severe diseases such as cerebral infarction and Parkinson's disease. At a first step for finding a clinically effective pharmaceutical agent, generally, an approach is now under way for finding a compound suppressing the death of nerve cells for example culture cells of the nerve system by screening using various target protein molecules suspected to be involved in the course of neurodegeneration and then certifying the effect of such compound in such central diseases-mimetic models of animals such as mouse and rat. It is considered that the discovery of a new target protein may be an important step for the discovery of a new therapeutic agent. Up to now, a great number of genetic products suspected to be responsible for the course of neurodegeneration have been found by analyses using experimental model animals mainly having mimetic cerebral infarction state.

Herein, peroxisome proliferator-activated receptors (PPAR) found as proteins mediating the function of increasing cellular small organelle peroxisome involved in fat degradation are members of the superfamily of transcription factors and nuclear receptors employing glucocorticoid, estrogen, progesterone, thyroid hormone and fat-soluble vitamins as the ligands. A great number of research works so far have indicated that PPAR has very important functions in controlling many genetic expressions and is involved in numerous diseases. Accordingly, the relation of PPAR with the death of nerve cells has been drawing attention.

It has been elucidated until now that human PPAR includes at least three sub-types of α, γ and δ. Each of the PPAR sub-types forms a hetero-dimer with RXR, using 9-cis retinoic acid as the ligand and is responsible for the regulation of the expression of diverse genes with the PPAR responsible element (PPRE: 5'-AGGTCA-X-AGGTCA-3') in the promoter regions. During the binding of such ligands to the PPAR-RXR hetero-dimer, it is generally considered that the dissociation of the co-repressor and the association to the co-activator occur, leading to the exertion of the transcription activation potency.

PPARα is highly expressed in tissues with high levels of fatty acid utilization, such as liver, kidney, heart and gastrointestinal tract and plays a role of regulating the metabolism of fatty acids, particularly the oxidation of fatty acids. Additionally, it is anticipated from research works on PPARα knockout mice that PPARα is closely involved in the onset of insulin resistance with highly fatty diets.

It is known that PPARγ has an important role in differentiation into fat cells. It is known for example that differentiation into fat cells is induced by causing PPARγ expression in fibroblast cells and subsequent treatment with a thiazolidine derivative (thiazolidinedione: TZD) as a potent synthetic PPARγ ligand. Further, the thiazolidine derivative is an agent for ameliorating the insulin resistance of type 2 diabetes accompanied by obesity. PPARγ induces differentiation into fat cells when exposed to a relatively high concentration of the ligands, such as in the case of administration of the thiazolidine derivative, leading to the increase of small-type fat cells and the apoptosis of enlarged fat cells. Consequently, PPARγ works for the activation of insulin sensitivity. When exposed to a relatively low concentration of the ligands such as in the case of a load of highly fatty diet (HF) in the absence of the thiazolidine derivative, it is considered that PPARγ works for the enlargement of fat cells, fat accumulation and insulin resistance.

Alternatively, PPARδ is expressed in diverse tissues such as brain, liver, kidney, spleen, fat, skeletal muscle, digestive tube, skin and placenta. It is reported that PPARδ has functions in fat cell differentiation, cerebral functions and epidermal differentiation. About 90 % of PPARδ knockout mice are dead at viviparity. Even when such mice are born, poor growth is observed throughout the viviparity to post-delivery, compared with the wild type. Furthermore, it is observed that their brains are small in proportion to the body sizes and myelin formation in the corpus callosum is abnormal. Additionally, epidermal hypertrophy is significantly enhanced in the knockout mice. As described above, it is suggested that PPARδ is closely involved in development, lipid metabolism, cerebral myelin formation, and epidermal cell growth. Still additionally, some reports tell about the activation of the cholesterol reverse transport system, the improvement of the composition ratio of lipoproteins and the decrease of neutral fat, with PPARδ selective agonists. PPARδ is a sub-type abundantly expressed in brain but almost no physiological role of PPARδ in brain has been elucidated.

The relation between the role of each of these PPAR sub-types and the neurodegenerative diseases has not yet been fully elucidated. However, so far, reports suggest that, regarding PPARγ which has been relatively increasingly elucidated, compounds with PPARγ agonist activities have some therapeutic effects on cerebral infarction and Parkinson's disease in animal models. For example, S. Sundararajan et al. report that PPARγ agonists are effective in rat models of cerebral infarction (S. Sundararajan, W.D. Lust, D.M.D. Landis and G.E. Landreth, Soc. Neurosci. Abstr. 26 (2000), p1808. PPAR gamma agonists reduce ischemic injury and immunoreactivity against inflammatory markers in rats.)

Further, S. Uryu et al. report that troglitazone suppresses the cellular death of cerebellar granule neurons and tells about an expectation of its use as a suppressing agent for the death of nerve cells. However, it is not clearly shown that PPARγ agonists have a general relation with the suppression of the death of nerve cells. (Shigeko Uryu, Jun Harada, Marie Hisamoto and Tomiichiro Oda. Brain Research 924 (2002) p229-236. Troglitazone inhibits both post-glutamate neurotoxicity and low-potassium-induced apoptosis in cerebellar granule neurons.)

Additionally, T. Breidert et al. report that one PPARγ agonist, pioglitazone, protectively works against the neurodegeneration of a mouse model of MPTP-induced Parkinson's disease (T. Breidert, J. Callebert, M.T. Heneka, G. Landreth, J.M. Launay and E.C. Hirsch. Journal of Neurochemistry 82 (2002) p615-624. Protective action of the peroxisome proliferator-activated receptor-gamma agonist pioglitazone in a mouse model of Parkinson's disease.) This may be ascribed to the anti-inflammatory effect of the PPARγ agonist.

WO0249626A2 discloses a therapeutic method for neurodegenerative diseases such as cerebral infarction and Parkinson's disease with PPARγ agonist.

Additionally, WO0213812A1 discloses a therapeutic method for neurodegenerative diseases and inflammatory diseases with PPARγ agonist. It specifies cerebral infarction, Parkinson's disease and Alzheimer's disease as the neurodegenerative diseases and also discloses that a PPARγ/PPARδ dual agonist has the same therapeutic effect. The specification however never includes any reference to the effect of the PPARδ agonist alone. The specification simply describes that the effect of the PPARγ agonist with low sub-type selectivity is due to the effect of the PPARγ/PPARδ dual agonist but never clearly verifies or discriminates the action of the PPARδ agonist from the action of the PPARγ agonist.

Herein, the possibility of a number of severe adverse actions of thiazolidinedione-series compounds such as rosiglitazone and pioglitazone as typical PPARγ agonists has been suggested. Among them, an adverse action of water retention is found at clinical tests of the pharmaceutical agents as therapeutic agents of type 2 diabetes and at various animal experiments (Sood V, Colleran K, Burge MR. Diabetes Technol Ther 2 (2000) p429-440. Thiazolidinediones: a comparative review of approved uses.) The enhancement of water retention often leads to cerebral edema and draw concerns about the exacerbating action of cerebral infarction. Therefore, the development of these PPARγ agonists as therapeutic agents of cerebral infarction may involve essential difficulty.

On the other hand, currently, no report tells that PPARδ has a direct relation with the onset of neurodegenerative diseases such as cerebral infarction and Parkinson's disease. No report has been known, telling a relation with edema. Nonetheless, some reports exist suggesting that PPARδ has a relation with apoptosis and inflammatory cellular death.

For example, T. Hatae et al. report apoptosis induction by the introduction of an expression vector of the prostacyclin synthase gene in human fetus kidney-derived 293 cell and activation of PPARδ (Toshihisa Hatae, Masayuki Wada, Chieko Yokoyama, Manabu Shimonishi and Tadashi Tanabe. Prostacyclin-dependent Apoptosis Mediated by PPARδ. The Journal of Biological Chemistry 276 (2001) pp.46260-46267).

Furthermore, WO0107066A1 describes that PPARδ inhibitors when administered inhibit the course of forming foam cell from macrophage and can therefore therapeutically treat various vascular diseases. The specification exemplifies cerebral stroke and Alzheimer's disease as various vascular diseases to be possibly treated. However, the specification simply discloses that PPARδ expressed in macrophage induces inflammatory reactions so that PPARδ is a factor exacerbating these diseases. In contrast, the role of PPARδ expressed in cells of the central nervous system or the possibility of PPARδ agonist as a therapeutic agent is absolutely never described.

### Disclosure of the Invention

Research works so far suggest that some of compounds with PPARγ agonist activities have a function to suppress neurodegenerative progress in cerebral infarction and the like. Since individual compounds with PPARγ agonist activities have more or less agonist activities simultaneously for PPARα and PPARδ in many cases, however, the relation between the agonist activity for each of the PPAR sub-types and the therapeutic effect on neurodegenerative diseases has not yet been fully elucidated. No report tells that a PPARγ agonist itself directly suppresses the death of neuron cells. Rather, an effect of indirectly suppressing nerve cell death via the essential anti-inflammatory action of such PPARγ agonist is highly possible. This suggests that the therapeutic effect of PPARγ agonist is limited only to the therapy of the inflammatory stage of neurodegenerative diseases involving inflammation. Additionally, it has been known so far that many of PPARγ agonists cause severe adverse actions such as water retention. Because cerebral edema is highly possibly exacerbated during the therapy of the cerebral nerve system, the possibility makes the application to clinical practice very tough. Therefore, it is highly demanded to select a compound capable of directly suppressing the death of neuron cells and effective for therapeutic treatment of various, diverse nerve diseases without any adverse actions such as water retention and to provide a great method applicable to clinical practice. It is an object of the present invention to overcome such problems. Present research works made by the inventors have first elucidated that agonists specific to PPARδ are singly effective for neurodegenerative diseases such as cerebral infarction and Parkinson's disease. Further, the research works have elucidated that PPARδ agonist directly interacts with neuron cell to suppress the death of the cell. These results indicate that PPARδ agonist may be effective for more diverse neurodegenerative diseases, compared with PPARγ agonist.

During the course of research works about investigations for culture cells derived from the nerve system and the relation between the actions of various nerve toxins and the death of nerve cells, the inventors have first found a fact that PPARδ agonist significantly suppresses the death of nerve cells due to thapsigargin, MPP⁺, staurosporine and the like. Therefore, the inventors have assessed the efficacy of these PPARδ agonists in model animals of cerebral infarction. The inventors have verified that these PPARδ agonists actually have an action of suppressing the death of nerve cells at an ischemic state following cerebral infarction. Additionally, the inventors have verified that the PPARδ agonists resume the MPTP-induced decrease of intracerebral dopamine content in a model animal of MPTP-induced Parkinson's disease. Additionally, the inventors have found that compounds with PPARδ agonist activities are useful for the therapeutic treatment of neurodegenerative diseases such as cerebral infarction and Parkinson's disease and that an efficacious therapeutic agent therefor can be selected among such compounds.

Specifically, the invention relates to a therapeutic method for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, which comprises administering a therapeutic agent for neurodegenerative diseases, the therapeutic agent containing a PPARδ agonist as an active ingredient.

Additionally, the invention relates to a therapeutic agent for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, the therapeutic agent containing a PPARδ agonist as an active ingredient.

In other words, the invention relates to those described below.
(1) A therapeutic agent for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, which comprises a PPARδ agonist as an active ingredient.
(2) A therapeutic method for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, which comprises administering a pharmaceutical agent comprising a PPARδ agonist as an active ingredient.
(3) A therapeutic agent for cerebral infarction, which comprises a PPARδ agonist as an active ingredient.
(4) A therapeutic agent for Parkinson's disease, which comprises a PPARδ agonist as an active ingredient.
(5) A therapeutic method for cerebral infarction, which comprises administering a pharmaceutical agent comprising a PPARδ agonist as an active ingredient.
(6) A therapeutic method for Parkinson's disease, which comprises administering a pharmaceutical agent comprising a PPARδ agonist as an active ingredient.
(7) The therapeutic agent described above in (1), (3) or (4), wherein the PPARδ agonist is a PPARδ agonist specifically re-selected using the activity of suppressing cellular death as the marker.
(8) The therapeutic method described above in (2), (5) or (6), wherein the PPARδ agonist is a PPARδ agonist specifically re-selected using the activity of suppressing cellular death as the marker.
(9) The therapeutic agent described above in (1), (3) or (4), where the PPARδ agonist is L-165041 or GW501516.
(10) The therapeutic method described above in (2), (5) or (6), wherein the PPARδ agonist is L-165041 or GW501516.
(11) Use of a PPARδ agonist for the manufacture of a therapeutic agent for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders.
(12) Use of a PPARδ agonist for the manufacture of a therapeutic agent for cerebral infarction.
(13) Use of a PPARδ agonist for the manufacture of a therapeutic agent for Parkinson's disease.
(14) The use described above in any of (11) through (13), wherein the PPARδ agonist is a PPARδ agonist
specifically re-selected using the activity of suppressing cellular death as the marker.
(15) The use described above in any of (11) through (13), wherein the PPARδ agonist is L-165041 or GW501516.
(16) An agent of suppressing the death of central nerve cell, which comprises a PPARδ agonist as an active ingredient.
(17) The agent of suppressing the death of central nerve cell as described above in (16), wherein the PPARδ agonist is L-165041 or GWW501516.

The invention is now described in detail hereinbelow.

The invention relates to a therapeutic agent for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, the therapeutic agent containing a PPARδ agonist as an active ingredient, as well as a therapeutic method thereof.

The PPARδ agonist in accordance with the invention is also referred to as PPARδ-like functioning agent or PPARδ-like acting agent and is a low molecular compound with various physiological actions by specifically binding to the PPARδ protein as a nuclear receptor to induce the modification of the structure, promoting the binding of the PPARδ-RXR complex to PPRE (peroxisome proliferator response element) and also promoting the expression of various genes with the PPRE sequence in the promoter regions.

Known compounds with the typical activities of the PPARδ agonist include for example L-165041 and GW501516.

It is reported that L-165041 (4-[3-[2-propyl-3-hydroxy-4-acetyl]phenoxy]propyloxyphenoxy acetic acid) has an activity binding to both PPARδ and PPARγ but its affinity to PPARγ (Ki 730 nM) is much weaker than its affinity to PPARδ (Ki 6 nM) (Mark D. Leibowitz et al., Activation of PPARδ alters lipid metabolism in db/db mice. FEBS Letters 473(2000) 333-336).

It is reported that GW501516 is a compound with a high affinity to PPARδ (Ki= 1.1 ± 0.1 nM) and exerts a high expression induction activity when the agonist activity is assayed using the GAL4-resposive reporter gene (EC₅₀ = 1.2 ± 0.1 nM) and that its effect involves a selectivity 1000-fold or more to PPARδ, compared with the selectivity to other PPAR sub-types (William R. Oliver, Jr. et al., A selective peroxisome proliferator-activated receptor δ agonist promotes reverse cholesterol transport. Proc. Natl. Acad. Sci. USA 98(2001) 5306-5311.)

The aforementioned reference information is supported by the preliminary experimental results made by the inventors. Specifically, the inventors verified about the selectivity of L-165041 and GW501516 to human and murine-derived individual PPAR sub-types on the basis of the assessment of the action of fusion proteins of GAL4-individual PPAR sub-types to activate transcription in the GAL4-responsive reporter gene that both the compounds had particularly high selectivity to human and murine PPARδ. It was also verified then that GW501516 had a higher selectivity and a higher expression induction activity than L-165041 (see Reference Example 1, Table 7).

Other than L-165016 and GW501516, numerous compounds have already been reported in WO2002100351, WO0200250048, WO0179197, WO0246154, WO0214291 and Japanese Patent Application No. 2001-354671 as existing agonists with relatively high activity to the subtype PPARδ. Additionally, Brown PJ et al. report compounds for example GW2433 (Brown PJ, Smith-Oliver TA, Charifson PS, Tomkinson NC, Fivush AM, Sterrnbach DD, Wade LE, Orband-Miller L, Parks DJ, Blanchard SG, Kliewer SA, Lehmann JM and Willson TM., Chem. Biol. (1997), p909-918. Identification of peroxisome proliferator-activated receptor ligands from a biased chemical library).

A person with ordinary experimental skills in the art can readily reselect a compound with a neuroprotective action according to the following selective method using culture cells, among these compounds.

The selective method of PPARδ agonist includes for example the following methods. But these methods are only illustrated. Thus, the selective method is not specifically limited to these methods. A person with ordinary skills in the art can readily optimize the details of these methods to develop and carry out the resulting method.

The reporter gene assay method for selecting PPAR agonists is known. For example, one hybrid reporter gene assay is reported, which employs the Gal4 transcription system of yeast and the Gal4-PPARδ fusion protein. (Lehman JM, More LB, Smith Oliver TA, Wilkinson WO, Willson TM, & Kliewer SA. An antidiabetic thiazolidinedione is a high affinity ligand for peroxisome proliferator-activated receptor γ (PPARγ). J. Biol. Chem. 270 (1995) 12953-12956.) The action of PPARδ to drive the yeast transcription system can be evaluated by the method.

As shown in the following references, PPAR agonists can be selected by reproducing the biological transcription system using a reporter assay system utilizing PPRE as the recognition response sequence of PPARδ and the full-length PPARδ.

(Dreyer C, Krey G, Keller H, Givel F, Helftenbein G, & Wahli W. Control of the peroxisomal β-oxidation pathway by a novel family of nuclear hormone receptors. Cell 68 (1992) 879-887.)

(Kliever SA, Forman BM, Blumberg B, Ong ES, Borgmeyer U, Mangelsdorf DJ, Umesono K, & Evans RM. Differential expression and activation of a family of murine peroxisome activated-activated receptors. Proc. Natl. Acad. Sci., USA 91 (1994) 7355-7359.)

(Devchand PR, keller H, Peters JM, Vazquez M, Gonzalez FJ, & Wahli W. The PPAR α-leukotriene B4 pathway to inflammation control. Nature 384 (1996) 39-43.)

(Forman BM, Chen J, & Evans RM. Hypolipidemic drugs, polyunsaturated fatty acids, and eicosanoids are ligands for peroxisome proliferator-activated receptors a and d. Proc. Natl. Acad. Sci., USA 94 (1997) 4312-4317.)

(Basu Modak S, Braissant O, Escher P, Desvergne B, Honegger P, & Wahli W. Peroxisome proliferator-activated receptor β regulates acyl-CoA synthetase 2 in reaggregated rat brain cell cultures. J. Biol. Chem. 274 (1999) 35881-35888.)

(He TC, Chan TA, Vogelstein B, & Kinzler KW. PPARδ is an APC-regulated target of nonsteroidal anti-inflammatory drugs. Cell 99 (1999) 353-345.)

For constructing a reporter assay system, generally, a transcription regulation region conjugated with a promoter of a known sequence which can express constitutively the PPAR responsible element (PPRE: 5'-AGGTCA-X-AGGTCA-3') in the promoter region is constructed. Then, a DNA construct artificially conjugated with a reporter gene is conjugated downstream the transcription regulation region. Additionally, an expression vector with the PPAR gene conjugated with a known gene promoter is constructed. These DNA constructs are introduced in appropriate culture cells (for example, CV-1 cell). Among various compounds then added, a compound capable of significantly increasing the expression level of the reporter gene is selected. As such known gene promoters, for example, promoters of SV40 virus initial gene and CMV IE gene may be used. However, the known gene promoters are not limited to them. A transcription regulation region with a general expression activity may be used satisfactorily. Such DNA construct can readily be constructed by a person with ordinary experimental skills in the art.

As to a sequence site essential for the transcription regulation of PPARδ and the like, an artificial DNA construct with plural such essential sequences in tandem repeat can be prepared by routine recombinant DNA experimental procedures. By using such artificial construct in replace of the natural sequence, the transcription-inducing activity of the transcription regulation region may sometimes be enhanced.

So as to accurately assay the PPARδ agonist activity, the method described above can be further improved to more accurately imitate the biological state. It is indicated that in biological organisms, PPARδ forms a heterodimer with RXR and further interacts with a co-activator of PPARδ, to exert its transcription activity. Therefore, the action of a compound with a PPARδ agonist activity to drive the PPARδ transcription system in a state more closely imitating the biological state can be evaluated by conjugating genes encoding these proteins or proteins functionally equivalent to the individual proteins to an expression vector to introduce the genes into cells. The individual genes may be introduced in one vector or different vectors. As the RXR gene, there may be used for example human RXRα gene (GenBank Accession No. NM_002957). As the co-activator, for example, human CBP gene (GenBank Accession No. U47741) and human SRC-1 gene (GenBank Accession No. U40396) may be used.

As the method for introducing the DNA construct carrying the PPAR reporter gene and the PPARδ gene, transformation method by any of general calcium phosphate method, liposome method, lipofectin method and electroporation method may satisfactorily be used with no specific limitation. More preferably, electroporation method is used.

Among compounds with PPARδ agonist activity as selected by the methods, a compound with preferable properties such as great neuroprotective action can be reselected. Specifically, a PPARδ agonist-derived test substance is added together with a certain neurotoxic substance to a cell for culturing, to count viable cells and compare the count with a cell count when only the neurotoxic substance is added to the cell, as carried out in a series of experiments (Example 1 through Example 6) made by the inventors. Through interactions of test substances, a test substance significantly suppressing the death of nerve cell as caused by a compound inducing certain neurotoxicity in a cell derived from the nerve system to reduce the lethal influence and increase the survival rate of the cell is then selected.

The entirety of the molecular mechanism of the death of nerve cell due to cerebral infarction has not yet been fully identified. By using for example thapsigargin as a substance inducing neurotoxicity, a person with ordinary experimental skills in the art can readily construct an assay system in a mimetic manner, using a culture cell for selecting a more optimized compound as an active ingredient of a therapeutic agent for cerebral infarction and the like. Thapsigargin is known as a potent inhibitor against calcium pump in sarcoplasmic reticulum (SR) and endoplasmic reticulum. Calcium is widely used for controlling cellular response, while calcium pump plays a very important role in maintaining cellular homeostasis. A report suggests that in the ischemic state at the acute stage of cerebral infarction, the disorder of intracellular calcium concentration in the neuron in the vicinity of cerebral infarction is one cause of cellular death.

It is considered that the concentration of thapsigargin for use in the assay system is a concentration of 1 nM or more to 1 µM or less, more preferably about 100 nM. The timing of adding thapsigargin is satisfactorily before or after the addition of test compounds. More preferably, thapsigargin is added 2 hours after the addition of test compounds. The activity of test compounds for protecting nerve cells in the assay system can be determined by any of the following methods, after overnight culturing in the concurrent presence of thapsigargin and test compounds. Any of the methods can readily be carried out by using any of commercially available kit products by a person with ordinary experimental skills in the art.
(1) Assaying viable cells by MTT assay.
(2) Assaying dead cells by LDH assay.
(3) Detecting apoptosis by caspase-3/7 assay.

When a certain test compound is added together with thapsigargin to cells in the assay system for culturing and when the significant increase of the survival rate of the cell is observed compared with the case of adding thapsigargin alone, the test compound possibly is the active component of a pharmaceutical agent effective for the therapeutic treatment of neurodegenerative diseases such as cerebral infarction.

As the culture cell for use in the assay system, a culture cell retaining the properties of human nerve cell is preferable. Cells of human neuroblastoma-derived established cell lines are preferably used. SH-SY5Y cell is more preferably used. It is considered that the use of the cell allows the establishment of an in vitro assay system of cellular death, which is similar to the mechanism of the death of nerve cell. Actually, both the compounds L-165041 and GW501516 with effects on the suppression of cellular death in the assay system significantly exert the effect on the suppression of cellular death in cerebral infarction models in vivo. Accordingly, a very good relation between the in vitro and in vivo effects is verified.

The entirety of the molecular mechanism of the death of cellular cell due to Parkinson's disease has not yet been fully elucidated. By using for example MPP⁺ (1-methyl-4-phenylpyridinium ion) as a substance triggering neurotoxicity, a cell system using a culture cell so as to select an optimized compound as an active ingredient in a therapeutic agent for Parkinson's disease can be constructed in a mimetic manner. One neurotoxin 1-methyl-4-phenyl-1,2,3,6tetrahydropyridine (MPTP) is known to induce Parkinsonism in humans and other primates and has drawn attention from the standpoint of the relation with the onset mechanism of Parkinson's disease. It is understood that after MPTP is incorporated in brain, MPTP is metabolized into MPP⁺ (1-methyl-4-phenylpyridinium ion) with monoamine oxidase B (MAOB) in astrocytes. MPP⁺ is incorporated into dopamine neurons with a dopamine transporter existing in the cell membrane of dopaminergic neurons. Further, the MPP⁺ incorporated in the dopamine neurons strongly inhibits the complex I in the electron transmission system in mitochondria, to exert its cellular toxicity and thereby induce symptoms similar to Parkinsonism. Because of such reasons, currently, endogenous or exogenous MPTP-analogous substances are suspected as substances causing Parkinson's disease. Currently, various research works are under way in various fields to verify whether or not MPTP-analogous substances selectively inhibiting dopaminergic neurons are contained in foods. Additionally, the possibility of a pharmaceutical agent reducing the neurotoxicity of MPP⁺ as a therapeutic agent for Parkinson's disease is promising.

It is considered that the concentration of MPP⁺ for use in the assay system is a concentration of 100 nM or more to 10 mM or less, preferably about 3 mM. The timing of adding MPP⁺ is satisfactorily before or after the addition of test compounds. More preferably, MPP⁺ is added 2 hours after the addition of test compounds. The activity of test compounds for protecting nerve cells in the assay system can be determined by any of the following methods, after overnight culturing in the concurrent presence of MPP⁺ and test compounds. Any of the methods can readily be carried out by using any of commercially available kit products, by a person with ordinary experimental skills in the art.
(1) Assaying viable cells by MTT assay.
(2) Assaying dead cells by LDH assay.
(3) Detecting apoptosis by caspase-3/7 assay.

When a certain test compound is added together with MPP⁺ to cells in the assay system for culturing and when the significant increase of the survival rate of the cell is observed, compared with the case of adding MPP⁺ alone the test compound is possibly the active component of a pharmaceutical agent effective for the therapeutic treatment of Parkinson's disease.

As the culture cell for use in the assay system, a culture cell retaining the properties of the dopaminergic nerve cell in human mesencephalic nigra is ideally preferable. Because a considerable part of the pathway causing cellular death is possibly due to the mechanism common to overall cells of the nerve system, cells of relatively readily handleable human neuroblastoma-derived established cell lines are now used instead. Cells of any human neuroblastoma-derived established cell line may be used. More preferably, SH-SY5Y cell can be used. It is considered that the use of the cell allows the establishment of an in vitro assay system of cellular death, which is similar to the mechanism of the death of nerve cell due to Parkinson's disease. Actually, both the compounds L-165041 and GW501516 with effects on the suppression of cellular death in the assay system significantly exert effects on the suppression of cellular death in an in vivo model of Parkinson's disease. Therefore, a very good correlation between the *in vitro* and in vivo effects is verified.

By using for example staurosporine instead of thapsigargin and MPP⁺ as a substance inducing neurotoxicity, a person with ordinary experimental skills in the art can readily construct an assay system using a culture cell for selecting an optimized compound as an active ingredient of a therapeutic agent for various neurodegenerative diseases in a mimetic manner. Staurosporine is one of microbial alkaloids generated by *Streptomyces staurosporeus* and is known as a non-specific inhibitor interacting with highly homologous catalyst regions among various numerous protein kinases. Although the entirety of the detailed molecular mechanism and the relation thereof with the diseases have not yet been fully elucidated, it is known that staurosporine is capable of widely inhibiting protein kinases having important functions in intracellular information transduction, leading to the death of nerve cells. For example, the disorder of the cellular information transduction pathway in the neuron in the vicinity of cerebral infarction lesion at the ischemic state at the acute stage of cerebral infarction is suggested as one cause triggering cellular death. Such disorder of intracellular information transduction may be converged into a mechanism common to numerous neurodegenerative diseases, which will lead to cellular death.

It is considered that the concentration of staurosporine for use in the assay system is a concentration of 1 nM or more to 1 µM or less, preferably about 150 nM. The timing of adding staurosporine is satisfactorily before or after the addition of test compounds. More preferably, staurosporine is added 2 hours after the addition of test compounds. The activity of test compounds for protecting nerve cells in the assay system can be determined by any of the following methods, after overnight culturing in the concurrent presence of staurosporine and test compounds. Any of the methods can readily be carried out by using any of commercially available kit products by a person with ordinary experimental skills in the art.
(1) Assaying viable cells by MTT assay.
(2) Assaying dead cells by LDH assay.
(3) Detecting apoptosis by caspase-3/7 assay.

When a certain test compound is added together with staurosporine to cells in the assay system for culturing, the test compound is possibly the active component of a pharmaceutical agent effective for the therapeutic treatment of neurodegenerative diseases such as cerebral infarction when the significant increase of the survival rate of the cell is observed, compared with the case of adding staurosporine alone. As the culture cell for use in the assay system, a culture cell retaining the properties of human nerve cell is preferable. Cells of human neuroblastoma-derived established cell lines are preferably used. SH-SY5Y cell is more preferably used. It is considered that the use of the cell allows the establishment of an *in vitro* assay system of cellular death, which is similar to the mechanism of the death of nerve cell. Actually, both the compounds L-165041 and GW501516 with actual effects on the suppression of cellular death in the assay system significantly exert the effect on the suppression of cellular death even in cerebral infarction models *in vivo*. Accordingly, a very good correlation between the *in vitro* and *in vivo* effects is verified.

The "PPARδ agonist specifically reselected using the activity of suppressing cellular death as a marker" in accordance with the invention is a compound obtained by reselecting a compound with a greater activity of suppressing cellular death by the method using a culture cell with thapsigargin, MPP+ or staurosporine added, from PPARδ agonists selected by known methods such as the reporter gene assay method. As the intensity of the activity of suppressing cellular death, the viable cell count assayed by the MTT assay method with the compound added at an optimal concentration within the range of 0.1 to 100 µM under the conditions described above is improved at least at 10 %, preferably at 30 % or more and more preferably at 50 % or more, compared with the case of never adding such compound.

The invention relates to a therapeutic method for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, as well as a therapeutic agent thereof containing a PPARδ agonist as an active ingredient.

The compound obtained by selecting a PPARδ agonist on the basis of the assessment method described above is useful for the therapeutic treatment and prophylaxis of the following diseases probably occurring via the degeneration of nerve cells.

Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders.

For using the PPARδ agonist in accordance with the invention as a pharmaceutical agent, the PPARδ agonist itself can be used as such. However, the PPARδ agonist may be formulated and used by known pharmaceutical methods. The therapeutic agent may be provided in any of forms including oral agent, parenteral agent or external agent. An administration route appropriate for the subject disease to be treated and an optimal dosage form for a subject person to be administered can be selected. For example, injections, infusions, syrups, tablets, granules, powders, troches, pills, pellets, capsules, microcapsules, suppositories, creams, ointments, aerosols, inhalation powders, liquids, emulsions, suspensions, enteric coating agents, sprays, eye drops, nasal drops and other appropriate dosage forms suitable for use may be possible and can be mixed with pharmaceutically acceptable, routine non-toxic carriers. If necessary, additionally, auxiliary agents, stabilizers, thickeners, coloring agents and flavor may be used. Such pharmaceutical formulations may be produced by routine methods using organic or inorganic various carriers common for formulation, including for example excipients (for example, sucrose, starch, mannit, sorbit, lactose, glucose, cellulose, talc, calcium phosphate, and calcium carbonate), binders (for example, cellulose, methyl cellulose, hydroxymethyl cellulose, polypropylpyrrolidone, gelatin, gum arabic, polyethylene glycol, sucrose and starch), disintegrators (for example, starch, carboxymethyl cellulose, hydroxypropyl starch, sodium hydrogen carbonate, calcium phosphate, and calcium citrate), lubricants (for example, magnesium stearate, aerosol, talc, and sodium lauryl sulfate), flavor (for example, citric acid, menthol, glycine and orange powder), preservatives (for example, sodium benzoate, sodium bisulfite, methyl paraben, and propyl paraben), stabilizers (for example, citric acid, sodium citrate and acetic acid), suspending agents (for example, methyl cellulose, polyvinylpyrrolidone, and aluminium stearate), dispersants ( for example, hydroxypropylmethyl cellulose), diluents (for example, water), base waxes (for example, cacao butter, white vaseline, and polyethylene glycol).

For producing these formulations, the pharmaceutical composition may satisfactorily contain pharmaceutically acceptable salts at an amount sufficient enough for the desired pharmaceutical effect to be exerted for the course or state of the disease. These salts may be used in the forms of pharmaceutical formulations in solid, semi-solid or liquid. Such pharmaceutically acceptable salts include routine non-toxic salts and specifically include for example metal salts such as alkali metal salts (for example, sodium salts or potassium salts) and alkali earth metal salts (for example, calcium salts or magnesium salts), inorganic acid-added salts (for example, hydrochloride salts, hydrobromide salts, sulfate salts and phosphate salts), organic carboxylic acid or sulfonic acid-added salts (for example, formate salts, acetate salts, trifluoroacetate salts, maleate salts, tartrate salts, fumarate salts, methanesulfonate salts, benzenesulfonate salts, and toluenesulfonate salts), and salts with basic or acidic amino acids (for example, arginine, aspartic acid and glutamic acid).

For administering the formulations to patients, these formulations are suitable for administration into nose and eye, external administration (topical), administration into rectum and lung (injections through nose or mouse), oral or parenteral (intraventricular, subcutaneous, intravenous and intramuscular) administration or inhalation. Administration via injections can be done by known methods such as intraarterial injections, intravenous injections and subcutaneous injections.

The dose of the therapeutic agent for the invention is an amount enough for the desired therapeutic effect to be exerted. The therapeutically effective amount of the compound is generally about 0.1 to 100 mg per day, preferably 1 to 16 mg per day for parenteral administration. The effective single dose is selected within a range of 0.001 to 1 mg per 1 kg • patient body weight, preferably 0.01 to 0.16 mg per 1 kg • patient body weight. Nonetheless, the dose may be modified in a manner depending on the body weight, age and symptoms of each patient to be treated and the dosing method and the like to be employed. A person with ordinary experimental skills in the art can appropriately select a more suitable dose on the basis of data from animal experiments and the like.

### Brief Description of the Drawings

### Best Mode for Carrying out the Invention

The invention is now described in detail in the following Examples. However, the invention is never limited to the Examples. The references in the related art as cited in the specification are all incorporated by reference in the specification.

### Example 1

### (1) Action of PPAR-• agonist on the suppression of cellular death in thapsigargin-induced cellular death model (neurodegenerative disease model)

### <Method>

### Assaying viable cells by MTT assay

SH-SY5Y cells were plated on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 × was added at 50 µl/well (for a blank, DMEM without serum alone was added). 2 hours later, DMEM without serum containing 600 nM thapsigargin was added at 20 µl/well. (Thapsigargin to a final 100 nM concentration) (For a

control, DMEM without serum alone was added.) 24 hours later, the viable cell count was calculated from the absorbance at 490 nm, using Celltiter 96 Aqueous one solution cell proliferation assay kit (Promega).

Table 1 shows the relation between the various concentrations of L-165041 or GW501516 added and the viable cell count thus determined. The results show the effect of the PPARδ agonist on the suppression of thapsigargin-induced cellular death.

**Table 1**

| | (•M) | Thapsigargin Thapsigargin | MTT assay A490 |
|---|---|---|---|
| L-165041 | 0 | - | 0.5536 ± 0.0060 |
| | 0 | + | 0.2156 ± 0.0043 |
| | 0.1 | + | 0.2176 ± 0.0036 |
| | 1 | + | 0.2158 ± 0.0034 |
| | 10 | + | 0.2436 ± 0.0015** |
| | 100 | + | 0.4405 ± 0.0065** |
| GW501516 | 0 | - | 0.7658 ± 0.0048 |
| | 0 | + | 0.2823 ± 0.0070 |
| | 0.1 | + | 0.2978 ± 0.0096 |
| | 1 | + | 0.3150 ± 0.0135 |
| | 10 | + | 0.3478 ± 0.0110** |
| | 100 | + | 0.2950 ± 0.0033** |

| | | | |
|---|---|---|---|
| Mean ± standard deviation (n = 4) | | | |
| ** P < 0.01 (one-way ANOVA followed by Dunnett's test vs thapsigargin-only treatment) | | | |

The individual assay values at A₄₉₀ in Table 1 are expressed in mean ± standard deviation from independent 4 experiments (n = 4). Among the control group with no treatment with any PPARδ agonist, the two groups of the thapsigargin-treated group and no thapsigargin-treated group were comparatively tested by Student's t-test. Significance at p < 0.01 was verified. For comparatively testing the individual groups treated with thapsigargin and various concentrations of the PPARδ agonist (L-165041 or GW501516), the difference in variance among the individual groups was verified by one-way ANOVA and subsequently tested by Dunnett's test. Significance at p < 0.01 is marked with asterisk (**) in Table 1.

In the assay system, cellular death is induced by adding thapsigargin (TG) as an inhibitor of CA²⁺ ATPase in endoplasmic reticulum to a neuroblastoma SH-SY5Y to give stresses to endoplasmic reticulum. Cellular death due to the stresses to endoplasmic reticulum is considered as a cause of the death of nerve cells at ischemic state. Accordingly, the assay system can be said as an assay system mimicking the death of nerve cells at ischemic state. The experiments indicated that L-165041 and GW501516 suppressed cellular death induced by thapsigargin (TG) in a concentration-dependent manner. (The cellular toxicity of GW501516 can be observed when GW501516 is at a concentration of 100 µM.)

### Example 2

### (2) Action of PPAR-• agonist on the suppression of cellular death in thapsigargin-induced cellular death model (neurodegenerative disease model)

### <Method>

### Assaying dead cells by LDH assay

SH-SY5Y cells were plated on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 x was added at 50 µl/well (for a blank, DMEM without serum alone was added). 2 hours later, DMEM without serum containing 600 nM thapsigargin was added at 20 µl/well. (Thapsigargin to a final 100 nM concentration) (For a control, DMEM without serum alone was added.) 24 hours later, the absorbance at 490 nm was assayed with a cytotoxicity detection kit (Roche) to assay the LDH activity. Table 2 shows the assay results of dead cells thus determined by LDH assay. An apparent correlation between the result of the viable cell count as determined by MTT assay in Example 1 and the LDH activity is shown. The above results clearly indicate that the PPARδ agonists are effective on the suppression of the thapsigargin-induced cellular death.

**Table 2**

| | ) | Thapsigargin | LDH release A490 |
|---|---|---|---|
| L-165041 | | - | 0.2258 ± 0.0049 |
| | | + | 0.6750 ± 0.0106 |
| | | + | 0.6758 ± 0.0183 |
| | | + | 0.6295 ± 0.0139 |
| | | + | 0.6029 ± 0.0135** |
| | | + | 0.2987 ± 0.0128** |
| GW501516 | | - | 0.1088 ± 0.0060 |
| | | + | 0.5413 ± 0.0171 |
| | | + | 0.6225 ± 0.0229 |
| | | + | 0.5750 ± 0.0212 |
| | | + | 0.3325 ± 0.0259** |
| | | + | 0.6675 ± 0.0328** |

| | | | |
|---|---|---|---|
| Mean ± standard deviation (n = 4) | | | |
| ** P < 0.01 (one-way ANOVA followed by Dunnett's test vs thapsigargin-only treatment) | | | |

### Example 3

### (3) Action of PPAR-• agonist on the suppression of cellular death in thapsigargin-induced cellular death model (neurodegenerative disease model)

### <Method>

### Detecting apoptosis by caspase-3/7 assay

SH-SY5Y cells were spread on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 × was added at 50 µl/well (for a blank, DMEM without serum alone was added). 2 hours later, DMEM without serum containing 600 nM thapsigargin was added at 20 µl/well well. (Thapsigargin to a final 100 nM concentration) (For a control, DMEM without serum alone was added.)

3 hours later, the caspase-3/7 activity was assayed with Apo-One homogenous Caspase-3/7 assay kit (Promega). Table 3 shows the results.

Since the PPARδ agonists L-165041 and GW501516 suppress the activity of caspase-3/7 activated during apoptosis induction, apparently, both these compounds directly or indirectly suppress apoptosis signal, so that these compounds are effective on the suppression of thapsigargin-induced cellular death.

**Table 3**

| | (µM) | thapsigargin | Caspase 3/7 activity RFLU |
|---|---|---|---|
| L-165041 | 0 | - | 147789 ± 5193 |
| | 0 | + | 702332 ± 14470 |
| | 0.1 | + | 732549 ± 24888 |
| | 1 | + | 652843 ± 8229 |
| | 10 | + | 554233 ± 23703** |
| | 100 | + | 242770 ± 5414** |
| GW501516 | 0 | - | 159016 ± 2356 |
| | 0 | + | 276057 ± 11115 |
| | 0.1 | + | 226861 ± 8501* |
| | 1 | + | 237165 ± 5248 |
| | 10 | + | 125951 ± 6465** |
| | 100 | + | 421972 ± 21782 |

| | | | |
|---|---|---|---|
| Mean ± standard deviation (n = 4) | | | |
| * p < 0.05 | | | |
| ** P < 0.01 (one-way ANOVA followed by Dunnett's test vs thapsigargin-only treatment) | | | |

### Example 4

### (1) Action of PPAR-• agonist on the suppression of cellular death in MPP⁺-induced cellular death model (Parkinson's disease model)

### <Method>

### Assaying viable cells by MTT assay

SH-SY5Y cells were spread on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 × was added at 50 µl/well (for a blank, DMEM without serum alone was added). 2 hours later, DMEM without serum containing 18 mM MPP⁺ was added at 20 µl/well. (MPP⁺ to a final 3 mM concentration) (For a control, DMEM without serum alone was added).

24 hours later, cell growth activity was assayed by measuring the absorbance at 490 nm, using Celltiter 96 aqueous one solution cell proliferation assay kit (Promega). Table 4 shows the results. The results apparently show that the PPARδ agonists are effective on the suppression of MPP⁺⁻induced cellular death.

In the assay system, MPP⁺ as an MPTP metabolite was added to a neuroblastoma SH-SY5Y, to inhibit the mitochondrial complex I to cause the generation of active oxygen and the inhibition of ATP synthesis, to induce cellular death. It is said that in the murine MPTP model commonly used as a model of Parkinson's disease, MPTP passing through the blood brain barrier is metabolized into MPP⁺, which exerts toxicity specific to murine nigra cell to induce nerve detachment. Therefore, the assay system can be said as an in vitro assay system mimicking the murine MPTP model. The experiments verify that L-165041 and GW501516 suppress cellular death induced by MPP⁺ in a concentration-dependent manner.

**Table 4**

| | (µM) | MPP⁺ | MTT assay A490 |
|---|---|---|---|
| L-165041 | 0 | - | 0.5448 ± 0.0041 |
| | 0 | + | 0.3392 ± 0.0048 |
| | 0.1 | + | 0.3744 ± 0.0067* |
| | 1 | + | 0.3935 ± 0.0083** |
| | 10 | + | 0.4799 ± 0.0078** |
| | 100 | + | 0.3181 ± 0.0122 |
| GW501516 | 0 | - | 0.7447 ± 0.0054 |
| | 0 | + | 0.3178 ± 0.0031 |
| | 0.1 | + | 0.3361 ± 0.0073 |
| | 1 | + | 0.3926 ± 0.0090** |
| | 10 | + | 0.3814 ± 0.0156** |
| | 100 | + | 0.2990 ± 0.0144 |

| | | | |
|---|---|---|---|
| Mean ± standard deviation (n = 4) | | | |
| * P < 0.05 | | | |
| ** p < 0.01 (one-way ANOVA followed by Dunnett's test vs MPP⁺-only treatment) | | | |

### Example 5

### (2) Action of PPAR-• agonist on the suppression of cellular death in MPP⁺-induced cellular death model (Parkinson's disease model)

### <Method>

### Assaying dead cells by LDH assay

SH-SY5Y cells were spread on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 × was added at 50 µl/well (for a blank, DMEM without serum alone was added). 2 hours later, DMEM without serum containing 18 mM MPP⁺ was added at 20 µl/well. (MPP⁺ to a final 3 mM concentration) (For a control, DMEM without serum alone was added.)

24 hours later, the dead cell count was calculated on the basis of the LDH activity assayed with a cytotoxicity detection kit (Roche). Table 5 shows the results. The results reveal that the PPARδ agonists are effective on the suppression of MPP⁺-induced cellular death. It was verified by the correlation between the assay results of cells in Example 4 and the results of dead cells by LDH assay from this experiment that L-165041 and GW501516 suppressed cellular death induced by MPP⁺ in a concentration-dependent manner. (Under the present assay conditions, it was observed that both L-165041 and GW501516 exerted cytotoxicity at the concentration of 100 µM.)

**Table 5**

| | (µM) | MPP⁺ | LDH release A490 |
|---|---|---|---|
| L-165041 | 0 | - | 0.2807 ± 0.0058 |
| | 0 | + | 0.4617 ± 0.0081 |
| | 0.1 | + | 0.3980 ± 0.0141* |
| | 1 | + | 0.3874 ± 0.0120** |
| | 10 | + | 0.3133 ± 0.0024** |
| | 100 | + | 0.6709 ± 0.0093 |
| GW501516 | 0 | - | 0.1610 ± 0.0052 |
| | 0 | + | 0.3772 ± 0.0187 |
| | 0.1 | + | 0.3633 ± 0.0153 |
| | 1 | + | 0.3222 ± 0.0207* |
| | 10 | + | 0.2981 ± 0.0154** |
| | 100 | + | 0.7969 ± 0.0251 |

| | | | |
|---|---|---|---|
| Mean ± standard deviation (n = 4) | | | |
| * P < 0.05 | | | |
| ** p < 0.01 (one-way ANOVA followed by Dunnett's test vs MPP⁺-only treatment) | | | |

### Example 6

### (3) Action of PPAR-• agonist on the suppression of cellular death in MPP⁺-induced cellular death model (Parkinson's disease model)

### <Method>

### Detecting apoptosis by caspase-3/7 assay

SH-SY5Y cells were spread on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 × was added at 50 µl/well (for a blank, DMEM without serum alone was added). 2 hours later, DMEM without serum containing 18 mM MPP⁺ was added at 20 µl/well. (MPP⁺ to a final 3 mM concentration.) (For control, DMEM without serum alone was added.)

3 hours later, the caspase-3/7 activity was assayed with Apo-One homogenous Caspase-3/7 assay kit (Promega). Table 6 shows the results.

Since the PPARδ agonists L-165041 and GW501516 both suppress the activity of caspase-3/7 in a concentration-dependent manner, the results indicate that both these compounds directly or indirectly suppress apoptosis signal, so that these compounds exert an effect on the suppression of MPP⁺-induced cellular death.

**Table 6**

| | (µM) | MPP⁺ | Caspase 3/7 activity RFLU |
|---|---|---|---|
| L-165041 | 0 | - | 185992 ± 3185 |
| | 0 | + | 1455497 ± 28684 |
| | 0.1 | + | 1430206 ± 30804 |
| | 1 | + | 1168920 ± 30554** |
| | 10 | + | 646736 ± 26366** |
| | 100 | + | 572152 ± 33695** |
| GW501516 | 0 | - | 114533 ± 5938 |
| | 0 | + | 933722 ± 18553 |
| | 0.1 | + | 847552 ± 36575 |
| | 1 | + | 621059 ± 26590** |
| | 10 | + | 798646 ± 54365 |
| | 100 | + | 398604 ± 55698** |

| | | | |
|---|---|---|---|
| Mean ± standard deviation (n = 4) | | | |
| * P < 0.05 | | | |
| ** p < 0.01 (one-way ANOVA followed by Dunnett's test vs MPP⁺-only treatment) | | | |

### Reference Example 1

### Action profiles and PPAR selectivities of L-165041 and GW501516 by in vitro receptor gene assay

Human PPARα cDNA, human PPARδ cDNA, human PPARγ cDNA, murine PPARα cDNA, murine PPARδ cDNA and murine PPARγ cDNA were individually introduced in the multi-cloning site of an expression vector pBIND (manufactured by Promega), to construct GAL4-human PPARα fusion protein-expressing vector pBINDhPPARα, GAL4-human PPARδ fusion protein-expressing vector pBINDhPPARδ, GAL4-human PPARγ fusion protein-expressing vector pBINDhPPARγ, GAL4-murine PPARα fusion protein-expressing vector pBINDmPPARα, GAL4-murine PPARδ fusion protein-expressing vector pBINDmPPARδ, and GAL4-murine PPARγ fusion protein-expressing vector pBINDmPPARγ. Together with a vector pG5luc (manufactured by Promega) for reporter gene expression, these expression vectors were individually introduced into African green monkey kidney-derived CV-1 cell using LipofectAMINE Reagent (manufactured by GIBCO BRL), to which a test compound L-165041 or GW501516 was added, for overnight culturing under conditions of 37°C and saturated humidity in the presence of 5 % CO₂. Then, firefly luciferase activity and Renilla luciferase activity were determined, using Dual-Luciferase Reporter Assay System (manufactured by Promega). The action of each PPAR sub-type to activate transcription was determined on the basis of the activity of firefly luciferase as a pG5luc reporter, using the activity of Renilla luciferase derived from pBIND as an internal standard. Table 7 shows the action profiles and PPAR selectivities of L-165041 and GW501516 by the in vitro receptor gene assay.

**Table 7**

| | Concentration (•M) of L-165041 for action exertion | Concentration (•M) of GW501516 for action exertion |
|---|---|---|
| Human PPAR-• | 10 | 1 |
| Human PPAR-• | 10 | >10 |
| Human PPAR-• | 0.1 | <0.01 |
| Murine PPAR-• | >100 | 10 |
| Murine PPAR-• | 10 | >10 |
| Murine PPAR-• | 0.1 | 0.01 |

### Example 7

### Action of PPAR-• agonist action in rat cerebral infarction model

Various compounds were evaluated in a rat cerebral infarction model. It was verified that L-165041 (PPAR-δ agonist) and GW501516 (PPAR-δ agonist) had potent actions to reduce cerebral infarction.

### <Method>

### Assessment of PPAR-δ agonist in cerebral infarction model (according to the Koizumi method)

L-165041 and GW501516 were dissolved in polyethylene glycol (PEG300). The pharmaceutical agents were given to male Wistar rats (age of 9 weeks), using an ALZET osmotic pressure mini-pump preliminarily filled aseptically with the pharmaceutical agents. On the day before a surgery for cerebral ischemia, a guide cannula was inserted in the right ventricule, namely at 0.8 mm backward from the coronal suture of the skull, and 1.5 mm unilaterally on the right side and in a 4.0-mm depth. Then, ventricular infusion was started at a flow rate of 1 µL/hour. The solvent was given to a control group in the same way. The infusion was sustained until the rats were sacrificed to death. As a cerebral infarction model, a middle cerebral artery-occluded reperfusion model according to the Koizumi's method was used. Specifically, a 4-0 nylon embolus with silicon coating and of a 19-mm length was inserted from the branch part of right common carotid artery toward internal carotid artery in the rats under anesthesia with halothane (initially at 4 % and retained at 1.5 %), to occlude the right middle cerebral artery. 90 minutes after ischemia, the nylon embolus was taken out under anesthesia again, for reperfusion. 24 hours after the reperfusion of cerebral ischemia, brain was resected, to prepare continuous coronary sections of a 2-mm thickness. The sections were stained with 2 % TTC (triphenyltetrazolium chloride) solution, to measure the damaged area of the brain and calculate the brain damage ratio.

Table 8 shows the results of measured cerebral damaged areas thus obtained. Table 9 shows the results of the assessment of the action of reducing cerebral infarction.

The results in Table 9 show that both L-165041 and GW501516 both have actions of reducing cerebral damages in a dose-dependent manner in the cerebral infarction model (according to the Koizumi's method). Further, GW501516 had a far greater action of reducing cerebral damages. The difference in the intensity of the action of reducing cerebral infarction between the two agents correlates well with the difference in the intensity of the action between the human and murine PPARδ agonists as measured by the in vitro reporter gene assay (Table 7). These results suggest that PPARδ agonists generally have an action of reducing cerebral infarction.

**Table 8 Action of PPAR-δ agonists in rat cerebral infarction model Lesions with cerebral infarction (area in % of damaged site in the 6 sections)**

| | Control group | L-165041 | | GW501516 | |
|---|---|---|---|---|---|
| µg/head/day | | 24 | 240 | 24 | 240 |
| Number of animals | (n = 8) | (n = 9) | (n = 9) | (n = 9) | (n = 9) |
| Total Total | 23.68 ± 1.96 | 22.68 ± 0.83 | 19.40 ± 1.98 | 18.74 ± 2.00 | 15.14 ± 2.20* |
| Cortex | 13.93 ± 1.23 | 12.70 ± 0.63 | 10.22 ± 1.29* | 9.39 ± 1.56 | 7.26 ± 1.67* |
| Subcortex | 9.74 ± 0.82 | 9.98 ± 0.30 | 9.19 ± 0.85 | 9.35 ± 0.65 | 7.88 ± 0.79 |

| | | | | | |
|---|---|---|---|---|---|
| Mean ± standard deviation | | | | | |
| * P < 0.05 (one way ANOVA followed by Dunnett's test vs control group) | | | | | |

**Table 9 Action of reducing cerebral infarction**

| | L-165041 | | GW501516 | |
|---|---|---|---|---|
| i.c.v. (•g/ head/day) | 24 | 240 | 24 | 240 |
| Reduction ratio of cerebral cortex damage | 9 % | 27 % | 33 % | 48 % |

### Example 8

### Action of PPAR-δ agonists in murine MPTP Parkinson's model

For the purpose of examining the neuroprotective action of PPAR• δ agonists in vivo, murine MPTP Parkinson's model was used.

### <Method>

### Assessment of PPAR-δ agonist in murine MPTP Parkinson's model

After male 9-week-old C57BL/6 mice non-fasted were anesthetized with pentobarbital (60 mg/kg, i.p.), the mice were fixed on a cerebral stereotactic apparatus, for fixing L cannula on the skull bone (at 0.0 mm to bregma, 1.2 mm lateral to midline, 2.5 mm ventral from skull) and subsequently embedding an ALZET osmotic pressure pump subcutaneously on a dorsal part. PPAR-δ agonists (L-165041 and GW501516) were dissolved in 30 % DMSO/saline, for filtration and sterilization. Subsequently, the pharmaceutical agents were aseptically charged in the osmotic pressure pump and then infused into the ventricule at 12 µg per day or 120 µg per day at a flow rate of 0.5 µL/hr. 2 days after the embedding of the pump, MPTP (20 mg/kg) was intraperitoneally administered twice at a 2-hour interval. 4 days after MPTP administration, the striate body was resected to assay the contents of dopamine (DA) and its metabolites 3,4-dihydroxyphenylacetic acid (DOPAC) and homovanillic acid (HVA) in the striate body by HPLC-ECD (high-performance liquid chromatography with electrochemical detection).

Table 10 shows the contents of DA and its metabolites per wet weight of striate body in the MPTP Parkinson's disease model as assayed in such manner. Additionally, Table 11 shows the resumption ratio of the contents of DA and its metabolites as calculated from the results in Table 10. These results indicate that the PPARδ agonists have an action of suppressing the decrease of the contents of DA and its metabolites DOPAC and HVA in the striate body due to MPTP in the murine MPTP Parkinson's disease model. The action of suppressing the decrease of the contents of DA and its metabolites DOPAC and HVA was greatly observed with L-165041 at a dose of 120 µg/head/day but not so greatly observed at a dose of 12 µg/head/day. The action was greatly observed with GW501516 at any of the doses. The difference in the intensity of the action of suppressing the decrease of DA content between these two agents correlates very well with the difference in the intensity of the agonist action between the human and murine PPARδ agonists as assayed by the in vitro reporter gene assay (Table 7). These results indicate that the actions of these compounds for suppressing the decrease of the contents of DA and its metabolites in striate body are generally ascribed to the PPAR-δ agonist activities.

**Table 10 Contents of DA and its metabolites per wet weight of striate body in MPTP Parkinson's disease model**

| | Dose (µg/head /day) | MPTP treat ment | Contents | | |
|---|---|---|---|---|---|
| | | | DA (ng/mg tissue) | DOPAC (ng/mg tissues) | HVA (ng/ mg tissue) |
| Normal | 0 | - | 15.59 ± 0.38 | 0.76 ± 0.03 | 1.31 ± 0.02 |
| Control | 0 | + | 5.01 ± | 0.29 ± 0.02 | 0.68 ± 0.03 |
| GW501516 | 12 | + | 7.41 ± 1.27 | 0.39 ± 0.06 | 0.82 ± 0.08 |
| | 120 | + + | 8.98 ± 1.20* | 0.44 ± 0.04* | 0.90 ± 0.05** |
| L-165041 | 12 | + | 5.06 ± 1.08 | 0.28 ± 0.05 | 0.66 ± 0.08 |
| | 120 | + | 7.81 ± 0.93* | 0.44 0.07 | 0.91 ± 0.10 |

| | | | | | |
|---|---|---|---|---|---|
| Mean ± standard deviation | | | | | |
| Normal group: n = 6; control group: n = 6; other individual groups: n = 7 | | | | | |
| * p < 0.05 (Student's t-test vs control) | | | | | |
| ** p < 0.01 (Student's t-test vs control) | | | | | |

**Table 11 Resumption ratios of contents of DA and its metabolites in MPTP Parkinson's disease model**

| | Dose (µg/head /day) | MPTP treat ment | Contents | | |
|---|---|---|---|---|---|
| | | | DA(%) | DOPAC (%) | HVA |
| Normal | 0 | - | 100.00 ± 3.56 | 100.00 ± 6.18 | 100.00 ± 3.05 |
| Control | 0 | + | 0.00± 3.67 | 0.00 ± 4.44 | 0.00 ± 4.27 |
| GW501516 | 12 | + | 22.67 ± 12.00 | 21.72 ± 11.73 | 21.42 ± 12.07 |
| | 120 | + | 37.53 ± 11.33* | 32.44 ± 9.17* | 33.93 ± 8.72** |
| L-165041 | 12 | + + | 0.42 ± 10.20 | -1.45 ± 10.89 | -4.65 ± 12.88 |
| | 120 | + + | 26.42 ± 8.83* | 32.22 ± 15.41 | 36.58 ± 16.68 |

| | | | | | |
|---|---|---|---|---|---|
| Mean ± standard deviation | | | | | |
| * p < 0.05 (Student's t-test vs control) | | | | | |
| ** p < 0.01 (Student's t-test vs control) | | | | | |

### Example 9

### (1) Action of PPAR-• agonist on the suppression of cellular death in staurosporine-induced cellular death model

Cellular death was induced by adding a protein kinase inhibitor staurosporine to a neuroblastoma SH-SY5Y to examine the effect of PPARδ agonists on the suppression of cellular death.

### <Method>

### Assaying viable cells by MTT assay

SH-SY5Y cells were plated on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 x was added at 50 µl/well (for a blank, DMEM without serum alone was added). 2 hours later, DMEM without serum containing 900 nM staurosporine was added at 20 µl/well. (Staurosporine to a final 150 nM concentration) (For a control, DMEM without serum alone was added.) 24 hours later, the viable cell count was calculated from the absorbance at 490 nm, using Celltiter 96 Aqueous one solution cell proliferation assay kit (Promega).

Table 1 shows the relation between the various concentrations of L-165041 or GW501516 added and the viable cell count thus determined. The results show the effects of PPARδ agonists (L-165041, GW501516) on the suppression of staurosporine-induced cellular death in a concentration-dependent manner.

**Table 12**

| | (µM) | Staurosporine | MTT assay A490 |
|---|---|---|---|
| L-165041 | 0 | - | 0.6607 ± 0.0022 |
| | 0 | + | 0.4249 ± 0.0086 |
| | 0.1 | + | 0.5523 ± 0.0209** |
| | 1 | + | 0.5532 ± 0.0056** |
| | 10 | + | 0.5167 ± 0.0098** |
| GW501516 | 0 | - | 0.6608 ± 0.0023 |
| | 0 | + | 0.4250 ± 0.0087 |
| | 0.1 | + | 0.5179 ± 0.0140** |
| | 1 | + | 0.5367 ± 0.0149** |
| | 10 | + | 0.5860 ± 0.0086** |

| | | | |
|---|---|---|---|
| Mean ± standard deviation (n = 4) | | | |
| ** P < 0.01 (one-way ANOVA followed by Dunnett's test vs staurosporine-alone treatment) | | | |
| ** p < 0.01 (Student's t-test vs control) | | | |

### Example 11

### (2) Action of PPAR-• agonist on the suppression of cellular death in staurosporine-induced cellular death model

### <Method>

### Assaying dead cells by LDH assay

SH-SY5Y cells were plated on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 x was added at 50 µl/well (for a blank, DMEM without serum alone was added). 2 hours later, DMEM without serum containing 900 nM staurosporine was added at 20 µl/well. (Staurosporine to a final 150 nM concentration) (For a control, DMEM without serum alone was added.) 24 hours later, LDH activity was assayed by measuring the absorbance at 490 nm, using Cytotoxicity detection kit (Roche). Table 2 shows the assay results of dead cells by LDH assay as determined in this manner. An apparent correlation is observed with the results of viable cells as determined by the MTT assay in Example 9. The results show that the PPARδ agonists have an effect on the suppression of staurosporine-induced cellular death.

**Table 13**

| | (µM) | Staurosporine | LDH release A490 |
|---|---|---|---|
| L-165041 | 0 | - | 0.1315 ± 0.0042 |
| | 0 | + | 0.6058 ± 0.0181 |
| | 0.1 | + | 0.5330 ± 0.0298 |
| | 1 | + | 0.5230 ± 0.0160 |
| | 10 | + | 0.5319 ± 0.0267 |
| GW501516 | 0 | - | 0.1315 ± 0.0042 |
| | 0 | + | 0.6058 ± 0.0181 |
| | 0.1 | + | 0.5430 ± 0.0264 |
| | 1 | + | 0.5201 ± 0.0313 |
| | 10 | + | 0.4025 ± 0.0188** |

| | | | |
|---|---|---|---|
| Mean ± standard deviation (n = 4) | | | |
| ** P < 0.01 (one-way ANOVA followed by Dunnett's test vs staurosporine-only treatment) | | | |

### Example 11

### (3) Action of PPAR-• agonist on the suppression of cellular death in staurosporine-induced cellular death model

### <Method>

### Detecting apoptosis by caspase-3/7 assay

SH-SY5Y cells were spread on a 96-well plate (70,000 cell/well in 100 µl DMEM low glucose 10 % fetal bovine serum) for overnight culturing, from which the culture medium was removed with an aspirator. Then, DMEM without serum was added at 50 µl/well. DMEM without serum containing a pharmaceutical agent (L-165041 or GW501516) at a concentration 2 x was added at 50 µl/well (for a blank, DMEM without serum alone was added). 2 hours later, DMEM without serum containing 900 nM staurosporine was added at 20 µl/well. (Staurosporine to a final 150 nM concentration) (For a control, DMEM without serum alone was added).

3 hours later, the caspase-3/7 activity was assayed with Apo-One homogenous Caspase-3/7 assay kit (Promega). Table 3 shows the results.

Since the PPARδ agonists L-165041 and GW501516 suppress the activity of caspase-3/7 activated during apoptosis induction, apparently, both these compounds directly or indirectly suppress apoptosis signal, so that these compounds are effective on the suppression of staurosporine-induced cellular death.

**Table 14**

| | (µM) | Staurosporine | Caspase 3/7 activity RFLU |
|---|---|---|---|
| L-165041 | 0 | - | 104281 ± 3078 |
| | 0 | + | 614433 ± 39923 |
| | 0.1 | + | 535404 ± 23301 |
| | 1 | + | 520732 ± 17273 |
| | 10 | + | 483017 ± 27259* |
| GW501516 | 0 | - | 104281 ± 3078 |
| | 0 | + | 614433 ± 39923 |
| | 0.1 | + | 586295 ± 18787 |
| | 1 | + | 463943 ± 12110** |
| | 10 | + | 420844 ± 13962** |

| | | | |
|---|---|---|---|
| Mean ± standard deviation (n = 4) | | | |
| * p < 0.05 | | | |
| ** P < 0.01 (one-way ANOVA followed by Dunnett's test vs staurosporine-only treatment) | | | |

### Industrial Applicability

In accordance with the invention, a compound with a protective action for nerve cell can be reselected by adding PPARδ agonist to a culture cell system where toxic substances such as thapsigargin, MPP⁺ and staurosporine are preliminarily allowed to react and reselecting a compound improving the survival rate. The compound selected by such method can be used as an active ingredient of a therapeutic agent for neurodegenerative diseases such as cerebral infarction and Parkinson's disease. Thus, the invention is very useful for research works for creating novel pharmaceutical agent.

## Claims

1. A therapeutic agent for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, which comprises a PPARδ agonist as an active ingredient.

2. A therapeutic method for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders, which comprises administering a pharmaceutical agent comprising a PPARδ agonist as an active ingredient.

3. A therapeutic agent for cerebral infarction, which comprises a PPARδ agonist as an active ingredient.

4. A therapeutic agent for Parkinson's disease, which comprises a PPARδ agonist as an active ingredient.

5. A therapeutic method for cerebral infarction, which comprises administering a pharmaceutical agent comprising a PPARδ agonist as an active ingredient.

6. A therapeutic method for Parkinson's disease, which comprises administering a pharmaceutical agent comprising a PPARδ agonist as an active ingredient.

7. The therapeutic agent according to claim 1, 3 or 4, wherein the PPARδ agonist is a PPARδ agonist specifically re-selected using the activity of suppressing cellular death as the marker.

8. The therapeutic method according to claim 2, 5 or 6, wherein the PPARδ agonist is a PPARδ agonist specifically re-selected using the activity of suppressing cellular death as the marker.

9. The therapeutic agent according to claim 1, 3 or 4, wherein the PPARδ agonist is L-165041 or GW501516.

10. The therapeutic method according to claim 2, 5 or 6, wherein the PPARδ agonist is L-165041 or GW501516.

11. Use of a PPARδ agonist for the manufacture of a therapeutic agent for Alzheimer's disease, Parkinson's disease, cerebral infarction, head injuries, cerebral hemorrhage, spinal injuries, multiple sclerosis, amyotrophic lateral sclerosis, Huntington's disease, diabetic or drug-induced peripheral nerve disorders or retinal nerve disorders.

12. Use of a PPARδ agonist for the manufacture of a therapeutic agent for cerebral infarction.

13. Use of a PPARδ agonist for the manufacture of a therapeutic agent for Parkinson's disease.

14. The use according to any of claims 11 through 13, wherein the PPARδ agonist is a PPARδ agonist specifically re-selected using the activity of suppressing cellular death as the marker.

15. The use according to any of claims 11 through 13, wherein the PPARδ agonist is a PPARδ agonist specifically re-selected using the activity of suppressing cellular death as the marker.

16. An agent of suppressing the death of central nerve cell, which comprises a PPARδ agonist as an active ingredient.

17. The agent of suppressing the death of central nerve cell according to claim 16, wherein the PPARδ agonist is L-165041 or GWW501516.
